# EUROPEAN PATENT APPLICATION

(11) **EP 0 939 068 A1**
(43) Date of publication of application: **01.09.1999**
(21) Application number: 96941884.7
(22) Date of filing: 17.12.1996
(51) Int. Cl.: C07B 63/00, B01D 9/02, C07C 15/24, C07C 7/14

(54) **METHOD FOR PURIFYING CRYSTALLINE SUBSTANCE**

(30) Priority: 26.12.1995 JP 33934195
(71) Applicant: KABUSHIKI KAISHA KOBE SEIKO SHO, Kobe-shi Hyogo-ken 651 (JP)
(72) Inventor: NAGAOKA, Koichi, Corporate Res. Labs. Kobe Steel, Kobe-shi, Hyogo-ken 651-22 (JP); YAMAMOTO, Koji, Corporate Res. Labs. Kobe Steel, Kobe-shi, Hyogo-ken 651-22 (JP); MOTOYUKI, Masahiro, Osaka Branch in Kobe Steel Ltd, Osaka-shi, Osaka 541 (JP)
(74) Representative: Blake, John Henry Francis
(86) International application number: JP9603668
(87) International publication number: WO9724302

(57) **Abstract**

A method for purifying a particular substance from a mixture containing the same employs steps (1) and (2a), or (2b), stated below to thereby recover the particular substance of high purity in high yield from the mixture by relatively easy operation:
(1) subjecting the mixture in the form of a liquid or a slurry in which part of the particular substance has crystallized to crystallization operation under high pressure to increase the amount of crystals of the particular substance to thereby obtain crystals enriched with the particular substance; and
(2a) dissolving the crystals enriched with the particular substance obtained in the above step in a solvent capable of well dissolving the crystals and subjecting a resultant solution to cooling crystallization to recrystallize the particular substance; or
(2b) rinsing the crystals enriched with the particular substance obtained in the above step with a solvent capable of dissolving the crystals.

## Description

### TECHNICAL FIELD

The present invention pertains to a purification method which makes it possible to obtain a particular crystalline substance of high purity in high yield from a mixture containing that substance. As an example, this method can be effectively used for obtaining 2, 6-dimethylnaphthalene from a mixture of dimethylnaphthalene isomers which is obtained in the form of coal tar or petroleum cut, or methylation of naphthalene or cyclization of a hydrocarbon.

### BACKGROUND ART

Conventional methods commonly used for purifying a particular substance by separating it from a mixture containing the substance include a distillation process based on a difference in boiling point and a cooling crystallization process based on a difference in solubility in a solvent at a given temperature. A pressure crystallization process based on pressure difference is another example of purification method proposed in recent years. Whichever method is used, however, it is difficult to purify a particular substance in high yield from feed stock (or raw material mixture) containing it at a low concentration in a single-stage process. While feed stock containing a particular substance at a relatively low concentration is passed through a plurality of processing stages to progressively increase the concentration of the desired substance in the commonly employed methods, various problems could arise during concentration and purification of the substance especially when the mixture contains any substance whose properties (e.g., boiling point and solubility in a solvent) are similar to those of the desired substance besides it.

Taking as an example purification of 2, 6-dimethylnaphthalene (hereinafter abbreviated as DMN), it is obtained by concentration of a coal tar or petroleum cut using a distillation process, or by methylation of naphthalene, or by cyclization of a hydrocarbon. Whichever process is used, 2, 6-DMN is obtained as a mixture containing a plurality of DMN isomers. There exist ten types of isomers of DMN. Since the boiling points of these isomers are close to one another, there are limitations in concentrating 2, 6-DMN by fractional distillation, and a maximum concentration of 2, 6-DMN achievable by it is 10 to 40%.

Although the applicant previously proposed a pressure crystallization process (Japanese Unexamined Patent Publication No. 63-275528) to provide an efficient method for purifying 2, 6-DMN, the concentration of 2, 6-DMN in feed stock must be about 50% or above in order that this process can be performed in an efficient manner. It is therefore difficult to obtain high-purity 2, 6-DMN by subjecting an ordinary feed stock whose 2, 6-DMN content is 10 to 40% in terms of concentration.

Thus, it is necessary to perform final purification by the pressure crystallization process after increasing the concentration of 2, 6-DMN to a level exceeding about 40% through preliminary concentration of the feed stock in order to obtain high-purity 2, 6-DMN by using the aforementioned pressure crystallization process. Method (A) shown below is one possible procedure for achieving this.

### (A) Purification method by the pressure crystallization process to be performed after concentration by the cooling crystallization process

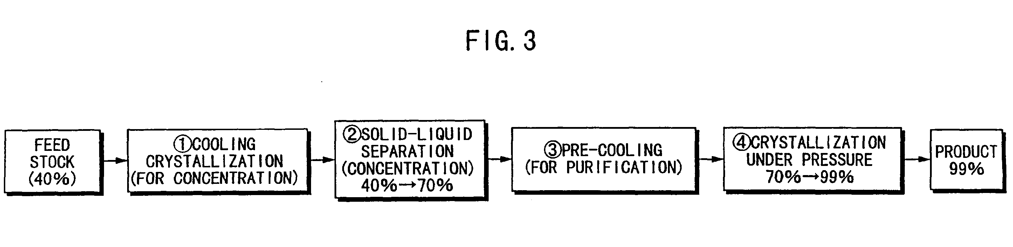

### Processing steps (see flowchart in FIG. 3):

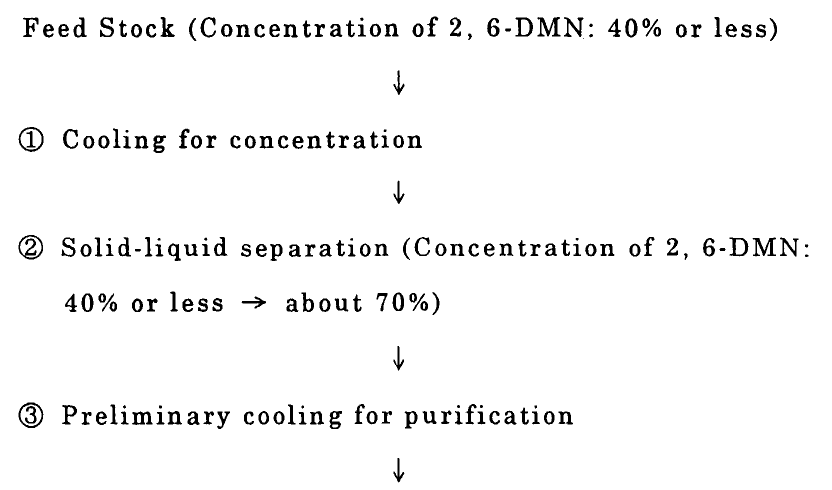

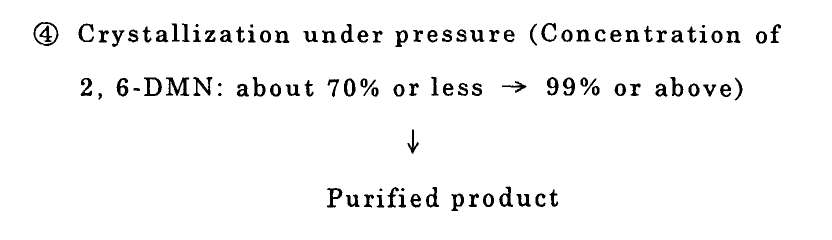

Feed stock is first cooled to convert it into the form of slurry containing crystals of a desired substance in step ①, and the slurry is treated by a solid-liquid separator, such as a centrifugal separator or a filter press, to separate and remove mother liquor containing a large amount of impurities in step ② to thereby increase the concentration of the desired substance up to such a level that is necessary for purification in a succeeding processing step. In step ③ a concentrated product obtained in step ② is cooled to form again a slurry in which part of the desired substance has crystallized, and in step ④, the slurry is subjected to pressure crystallization in which the amount of crystals of the desired substance is increased as a result of pressurization and the mother liquor containing a large amount of impurities is discharged to eventually obtain high-purity 2, 6-DMN.

Since the concentration of the desired substance within the slurry serving as feed stock for pressure crystallization is low (about 70%) in the aforementioned pressure crystallization process of step ④, it is necessary to keep a relatively low crystallization pressure for preventing precipitation of the impurities as much as possible and to remove the impurities which have adhered to surfaces of the crystals as much as possible by allowing sufficient sweating which occurs in a final stage of pressure crystallization in order to purify the desired substance up to such high purity as 99% or so in a single operation. Thus, the recovery of the desired substance significantly decreases in this process.

Although it would be possible to divide the pressure crystallization process into a plurality of stages to progressively increase the concentration of the desired substance such as 70% to 80% in a first stage, 80% to 90% in a second stage, and 90% to 99% in a final stage, for instance, the recovery of the desired substance significantly decreases in this method. This is because work efficiency is low and total loss given by multiplying losses in the individual stages increases in this method.

Method (B) below is another possible concentration and purification procedure:

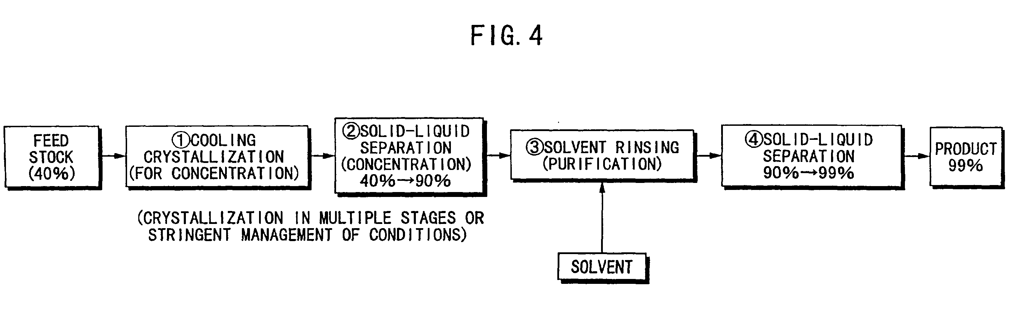

### (B) Method employing the cooling crystallization process for both concentration and purification Processing steps (see flowchart in FIG. 4):

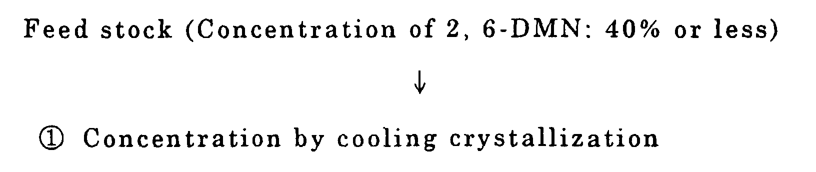

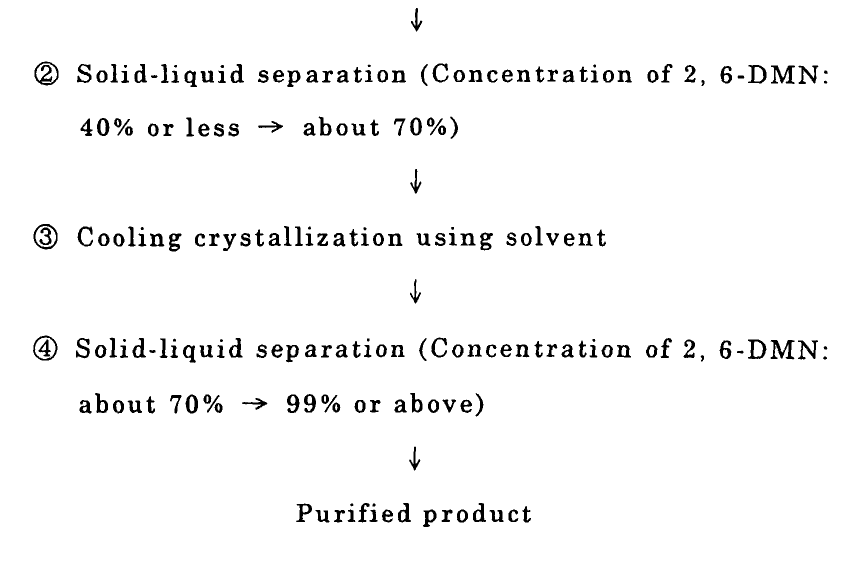

Specifically, feed stock is first cooled to convert it into the form of slurry containing crystals of a desired substance in step ①, and the slurry is treated by a solid-liquid separator, such as a centrifugal separator or a filter press, to separate and remove mother liquor containing a large amount of impurities in step ② to thereby increase the concentration of the desired substance up to such a level that is necessary for purification in a succeeding processing step in a manner similar to the above-described method (A). It is necessary to increase the concentration of the desired substance to a fairly high level in these steps of purification in order to perform final purification by cooling crystallization. Thus, it is essential to keep the amount of mother liquor (liquid content) which remains adhering to the crystals to about 10% in the solid-liquid separation process of step ② to make it possible to remove the impurities as much as possible.

It is therefore necessary in this process to allow sufficient sweating in a final stage of separation and sufficiently remove the impurities by using a solid-liquid separation method like filter press which can remove the mother liquor to a high degree of perfection. Since the amount of the desired substance discharged together with the mother liquor increases as a matter of course, the recovery of the desired substance decreases. Although it would be possible to divide the process of concentration by cooling crystallization into a plurality of stages to progressively increase the concentration of the desired substance such as 40% to 60% in a first stage, 60% to 80% in a second stage, and 80% to 90% in a final stage, for instance, the recovery of the desired substance significantly decreases in this method. This is because work efficiency is low and total loss given by multiplying losses in the individual stages increases in this method.

When concentration to a level of 90% has been accomplished, purification is performed by final cooling crystallization in step ③. When performing purification by using the cooling crystallization process, the concentration of the slurry (or the concentration of the crystals in the solid-liquid mixture) greatly varies as a result of small temperature changes if the concentration of the desired substance in the feed stock is high, making it difficult to handle the slurry and perform solid-liquid separation. It is therefore necessary to perform cooling crystallization after diluting the slurry by adding a solvent. An aromatic solvent like benzene or a kind of alcohol is used as solvent for 2, 6-DMN. Subsequently, the slurry in which the desired substance has precipitated is subjected to a solid-liquid separation process and the desired substance is obtained in the form of crystals in step ④. A centrifugal separation method may also be used for solid-liquid separation in this step.

There exists a common problem in the aforementioned methods (A) and (B), however. Specifically, it is not possible to sufficiently increase the concentration of the crystals in the slurry due to the need for increasing the ease of handling because either of the methods employs the cooling crystallization process for concentrating the desired substance and, as a consequence, it is not possible to sufficiently increase the recovery of the desired substance in the overall processing procedure. More particularly, the concentration of the crystals in the slurry that can be treated is limited to about 20% in the light of the ease of transport and handling and it is difficult to obtain a slurry with a higher crystal content in a single-stage concentration process and, therefore, the recovery of the desired substance naturally becomes low with concentration limitations serving as a rate-determining factor. In order to increase the recovery of the desired substance, it is necessary to perform more than once recovery and concentration of the desired substance dissolved in the mother liquor which is discharged in the concentration process. This results in complex operation and degradation in efficiency as well as the need for expansion of processing facilities.

The following problem occurs as well in the aforementioned method (A) when handling a desired substance having a high melting temperature. In order to concentrate a substance having a high melting temperature, such as 2, 6-DMN whose melting temperature is 110°C, by the cooling crystallization process, the temperature of its processing facility must be increased to a point higher than the melting temperature of the substance. This will result in not only complex control of the facility, such as heat insulation, but also difficult temperature control due to the need for performing purification by sweating at a high temperature as well as inability to sufficiently increase ultimate purity.

The present invention has been made in view of the problems of the prior art technology stated above. Accordingly, it is an object of the invention to provide a method which makes it possible to effectively perform concentration and purification of a particular substance from feed stock containing it at a low concentration by a relatively easy procedure.

### SUMMARY OF THE INVENTION

A method for purifying a crystalline substance of the invention is a method for purifying a particular substance from a mixture containing the same, the method being characterized by carrying out steps (1) and (2a), or steps (1) and (2b), as stated below:
(1) subjecting the mixture in the form of a liquid or a slurry in which part of the particular substance has crystallized to crystallization operation under high pressure to increase the amount of crystals of the particular substance to thereby obtain crystals enriched with the particular substance; and
(2a) dissolving the crystals enriched with the particular substance obtained in the above step in a solvent capable of well dissolving the crystals and subjecting a resultant solution to cooling crystallization to recrystallize the particular substance; or
(1) subjecting the mixture in the form of a liquid or a slurry in which part of the particular substance has crystallized to crystallization operation under high pressure to increase the amount of crystals of the particular substance to thereby obtain crystals enriched with the particular substance; and
(2b) rinsing the crystals enriched with the particular substance obtained in the above step with a solvent capable of dissolving the crystals.

A considerable amount of particular substance, which is a desired substance, is contained either in the mother liquor left after separating the particular substance formed by recrystallization in step (2a) or in the rinsing solution obtained in step (2b). Thus, in carrying out the aforementioned method, it is preferable to remove the solvent and supply a resultant residue for steps (1) and (2a), or steps (1) and (2b), mentioned above for reprocessing, because the yield of the desired substance can be further increased by doing so.

It is also preferable to return the solvent obtained by evaporating the mother liquor or the rinsing solution in the aforementioned steps as the solvent to be used in step (2a) or step (2b) for recycling, because it becomes possible to reduce solvent consumption by doing so.

This purification method can be effectively used as a method for separating and purifying 2, 6-DMN as a particular substance from a mixture 2, 6-DMN isomers, for instance. For this kind of application, an alcohol is preferable as the solvent capable of well dissolving the crystals for use in performing cooling crystallization while an aromatic solvent is preferable as the solvent capable of dissolving the crystals for use in rinsing the crystals.

### BRIEF DESCRIPTION OF THE DRAWINGS

FIG. 1 is a flowchart illustrating a concentration/purification method employed in this invention;
FIG. 2 is a flowchart illustrating another concentration/purification method employed in this invention;
FIG. 3 is a flowchart illustrating a conventional concentration/purification method; and
FIG. 4 is a flowchart illustrating another conventional concentration/purification method.

### BEST MODES OF CARRYING OUT THE INVENTION

As previously mentioned, a purification method employed in this invention adopts a pressure crystallization process for concentration of a particular substance to be performed in a first stage of purification and cooling crystallization using a solvent or solvent rinsing in a final stage of purification. Using a combination of these processes, the purification method has succeeded in eliminating the foregoing problems pointed out with respect to the prior art technology and obtaining the particular substance of high purity in high yield by relatively easy operation and procedure.

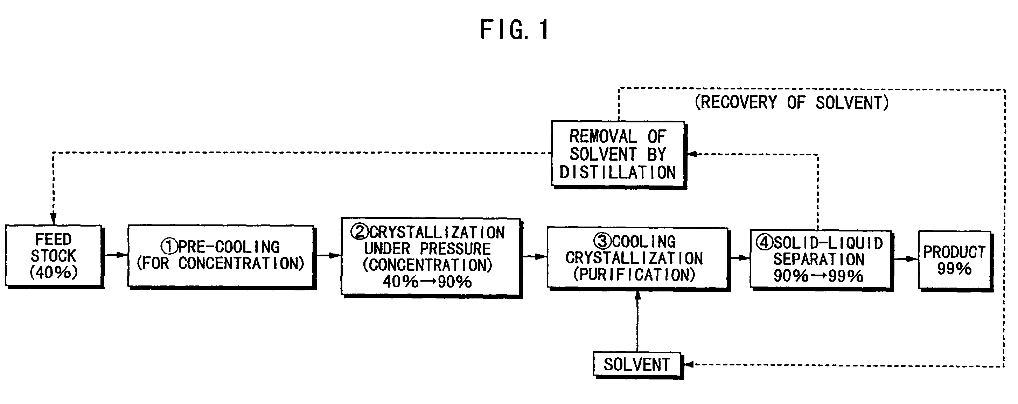

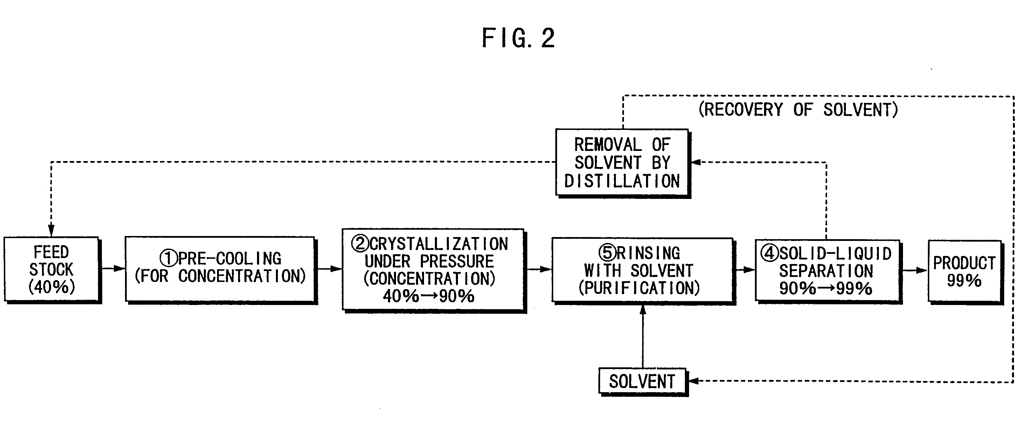

FIGS. 1 and 2 are flowcharts illustrating purification procedures employed in this invention, in which FIG. 1 shows the procedure to be followed in carrying out a method described in claim 1 while FIG. 2 shows the procedure to be followed in carrying out a method described in claim 4.

Referring first to the flowchart of FIG. 1, feed stock (raw material mixture) containing a desired substance at a given concentration is cooled to convert it into the form of slurry containing crystals of the desired substance in step ① in substantially the same way as the previously described conventional methods and, then, the slurry is concentrated by crystallization under pressure in step ②.

When the method in which the slurry is concentrated by the pressure crystallization process in this manner, no limitations are imposed on the concentration of the slurry in a high-pressure vessel. This means that, unlike the earlier-mentioned conventional approach employing the cooling crystallization process, it is not necessary to limit the concentration of the slurry to about 20% due to the need to ensure the ease of transport and handling and the concentration of the slurry can be sufficiently increased so that it becomes possible to significantly increase the recovery of the desired substance compared to the approach based on cooling crystallization. Furthermore, provided that concentration by crystallization under pressure is limited to about 90% in terms of the concentration of the desired substance, discharging of mother liquor may be stopped halfway without performing such operations as sweating, rinsing and compression in ordinary crystallization under pressure for the purpose of purification to the very end. As a result, the loss of the desired substance which occurs as it is mixed and discharged together with the mother liquor can be reduced and, thus, the recovery of the desired substance is further increased. Although high-precision control devices (for controlling temperature, pressure, the amount of discharged mother liquor, etc.) are required to reliably perform sweating, rinsing and compression for accomplishing purification by way of crystallization under pressure, control of the pressure crystallization process for the purpose of concentration can be greatly simplified, making it possible to simplify devices and mechanisms of pressure crystallization equipment.

There is another advantage in performing concentration by crystallization under pressure, which is described below. It is fairly difficult to purify the desired substance to such a high level of purity as 99% or over by a single-stage crystallization under pressure by using feed stock in which the concentration of the desired substance is about 50% or less. The purity of purified crystals obtained by crystallization under pressure depends on the amount and purity of the mother liquor remaining in the purified crystals. Since the concentration of impurities within the mother liquor becomes high when a low-concentration feed stock is subjected to crystallization under pressure, the purity of purified crystals can not be easily increased. Since the concentration of impurities within the mother liquor remaining in crystals may however be relatively high when using the pressure crystallization process for concentration up to a purity of about 90%, it becomes possible to reduce the amount of the desired substance discharged together with the mother liquor and thereby increase the recovery of the desired substance by discharging the mother liquor under slightly higher pressure than the pressure used for purification.

After concentrating the desired substance up to a point where its concentration reaches about 90% in step ②, the concentrated crystals are to be dissolved in a suitable solvent and subjected to cooling crystallization by using a conventional procedure in step ③ and, then, the desired substance can be obtained in the form of high-purity crystals through solid-liquid separation in step ④. Since the concentration of the desired substance in the concentrated crystals which are used as feed stock in the cooling crystallization process of step ③ has already been increased to about 90% in the pressure crystallization process of step ②, there is less load during the cooling crystallization process and the loss of the desired substance is reduced. Thus, it becomes possible to obtain the high-purity desired substance in high yield.

Although the cooling crystallization process using the solvent is employed in step ③ of the above-described method, a purification process by solvent rinsing of step ⑤ is adopted instead of step ③ in the method of FIG. 2. Specifically, a desired substance within mother liquor crystallizes and grows around seeds which are formed of the same desired substance in the pressure crystallization process of step ②, and what causes a reduction in the purity of the concentrated crystals is the impurities within the mother liquor which remains adhering to surfaces of the crystals as previously mentioned. Thus, it would be possible to obtain the desired substance as high-purity crystals from the concentrated crystals if the concentrated crystals is rinsed with a suitable solvent to thereby rinse out the mother liquor remaining on their surfaces and the liquid phase is removed by solid-liquid separation of step ④.

When the methods of FIGS. 1 and 2 are carried out, a fairly large amount of the desired substance is dissolved and mixed in the mother liquor or in a solvent-containing rinsing solution which are discharged in the solid-liquid separation of step ④. The desired substance is mixed in the mother liquor or the rinsing solution at a concentration of about 60 to 75% although the actual concentration varies depending on the type and amount of solvent used. Thus, it is preferable to return the mother liquor and the rinsing solution after removing off the solvent back to each successive lot of starting material and cyclically process them as shown by broken lines in FIGS. 1 and 2, because the recovery of the desired substance in the overall concentration and purification system can be increased by doing so.

It is also preferable to return the solvent removed in this process and recycle it as at least part of the solvent used in the cooling crystallization (purification) process of step ③ or in the solvent rinsing (purification) process of step ⑤ as shown by broken lines in FIGS. 1 and 2, because it becomes possible to reduce solvent consumption by doing so.

As described above, it has become possible to increase the concentration of the slurry without any problem by employing the pressure crystallization process for accomplishing concentration from the low-concentration feed stock having a concentration of the particular substance of about 40% to the concentration of the particular substance of about 90% to thereby increase the recovery of the desired substance and alleviate the load in later purification in this invention. Furthermore, it has become possible to recover the particular substance of high purity in high yield from the feed stock of a relatively low concentration by easy operation and procedure by employing the cooling crystallization process using the solvent or the solvent rinsing process which can efficiently perform purification from a high-concentration product by easy operation in the final purification process.

While specific operating conditions (temperature, pressure, etc.) applied to the pressure crystallization and cooling crystallization processes employed in this invention may be optimally determined as appropriate depending on the type of desired substance and impurities, for instance, the crystallization operating pressure for increasing the amount of crystals in step ① above should preferably be set within a range of 500 to 3,000 kg/cm², and more preferably 1,000 to 2,000 kg/cm². The reason for this is that the increase in the amount of crystals of the particular substance becomes insufficient if the crystallization pressure is less than 500 kg/cm², making it difficult to sufficiently increase the yield of the substance, while significant heat generation occurs due to pressurization if the crystallization pressure is excessively increased beyond 3,000 kg/cm², not only causing a problem in pressure crystallization but also making it necessary to provide the crystallization equipment with cooling means, which is not desirable from the viewpoint of the equipment and operation.

The type of solvent to be used for cooling crystallization or rinsing for purification may be determined by properly choosing it depending on the type of desired substance. If the desired substance is 2, 6-DMN, for example, examples of preferable solvents to be used for cooling crystallization in the aforementioned step (2a) are lower alcohol solvents such as methyl alcohol and ethyl alcohol, whereas examples of preferable solvents to be used for rinsing in the aforementioned step (2b) are aromatic solvents such as benzene, toluene and xylene.

While a typical example of a particular substance to be purified by applying the methods of this invention is 2, 6-DMN as already mentioned, the methods can be applied to any mixture if only it is of a type in which the particular substance first crystallizes as depicted in a phase diagram of a solution of the mixture containing the particular substance to be purified. Compounds of this kind other than 2, 6-DMN that can be cited as examples include aromatic compounds such as phenol, cresols, naphthalene, halogenated benzenes, aromatic compounds, alkylbenzenes and alkylnaphthalenes as well as heterocyclic compounds such as indole having nitrogen in a ring, for instance.

### PRACTICAL EXAMPLES

While the invention is now described in greater detail with reference to practical examples, it is not limited by the following practical examples but may be implemented upon making appropriate modifications thereto as long as such modifications comply with the spirit of the invention shown in the foregoing and following discussion, and any of these modifications fall within the technical scope of the invention. It is to be noted that the expression "%" used in the following discussion means "weight %".

### PRACTICAL EXAMPLE 1

Using a mixture formed of the following components obtained by a preliminary cooling crystallization process as feed stock:
- 2, 6-DMN content:: 40%
- 2, 7-DMN content:: 16%

Other DMN isomer content: 44% 100 kg of the same was heated to 48°C to achieve a slurry concentration of 20% and subjected to crystallization under high pressure to concentrate the 2, 6-DMN content. Crystals were formed while maintaining a pressure of 1,500 kg/cm² and a temperature of 70 °C for 3 minutes in this pressure crystallization and, subsequently, a 28-kg cake of crude crystals with a 2, 6-DMN content of 90% was obtained by forcing out mother liquor through a filter.

Next, the crude crystal cake was taken out of a pressure vessel and after adding ethyl alcohol measuring five times the weight of the crude crystal cake to it, the whole crude crystals were dissolved by heating up to 60 °C. Subsequently, a resultant solution was cooled down to 20°C to cause crystals of 2, 6-DMN to deposit and 18.2 kg (45.5% of the amount of 2, 6-DMN contained in the feed stock) of crystals having a 2, 6-DMN purity of 99% was obtained by performing solid-liquid separation using a centrifugal separator.

### PRACTICAL EXAMPLE 2

A 28-kg cake of crude crystals with a 2, 6-DMN content of 90% was obtained by using a mixture of DMN isomers having the same composition as practical example 1 described above as feed stock which was obtained by the preliminary cooling crystallization process, and performing concentration by crystallization under pressure in the same manner as practical example 1.

Next, the crude crystal cake was taken out of a pressure vessel and crushed, and after adding benzene measuring twice the weight of the crude crystal cake to it, the crude crystals were rinsed at 20°C by agitating a resultant mixture for a period of one hour. 18.0 kg (45.0% of the amount of 2, 6-DMN contained in the feed stock) of crystals having a 2, 6-DMN purity of 99% was obtained by subsequently performing solid-liquid separation using a centrifugal separator.

### PRACTICAL EXAMPLE 3

When the concentration and purification procedure of practical example 1 described above is executed, the mother liquor removed during solid-liquid separation performed by the centrifugal separator in the purification process contains a considerable amount of 2, 6-DMN. In this circumstance, the solvent within the mother liquor was removed and the recovered solvent was reused as a solvent for dissolving the crude crystals. Furthermore, a mixture weighing 9.8 kg with a 2, 6-DMN content of 71% was obtained as a residue left after removal of the solvent. This 9.8-kg mixture was mixed with 100 kg of the original feed stock (2, 6-DMN content: 42.8%) and a resultant mixture was subjected to concentration and purification in exactly the same manner as practical example 1. As a result, 20.0 kg (50.0% of the amount of 2, 6-DMN contained in the feed stock) of crystals having a 2, 6-DMN purity of 99% was finally obtained.

### COMPARATIVE EXAMPLE 1

Employing the earlier-mentioned conventional method (A) entitled "Purification method by the pressure crystallization process to be performed after concentration by the cooling crystallization process," 100 kg of feed stock having the same composition as used in practical example 1 was heated to 48°C to achieve a slurry concentration of 20%. When this slurry was subjected to solid-liquid separation by slight compression filtration, 28.6 kg of concentrated product with a 2, 6-DMN content of 70% containing a considerable amount of mother liquor entrapped in spaces between crystals was obtained.

This concentrated product was heated to 87 °C to achieve a slurry concentration of 20% and subjected to pressure crystallization and solid-liquid separation in a manner similar to practical example 1. Since the concentration of the desired substance within the mixture subjected to pressure crystallization was low (70%) in this case, it was necessary to sufficiently perform sweating, rinsing and compression during solid-liquid separation in order to ensure such a high level of purity as 99%. A large amount of mother liquor had to be discharged in the form of liquid phase, and crystals finally obtained with a 2, 6-DMN content of 99% weighed 10.6 kg (26.5% of the amount of 2, 6-DMN contained in the feed stock). The recovery of 2, 6-DMN was only about 60% of that achieved in practical examples 1 and 2, and 53% of that achieved in practical example 3. This means that this method is remarkably unsatisfactory in terms of the recovery of the desired substance.

### COMPARATIVE EXAMPLE 2

Employing the earlier-mentioned conventional method (B) entitled "Method employing the cooling crystallization process for both concentration and purification," 100 kg of feed stock having the same composition as used in practical example 1 was heated to 48 °C to achieve a slurry concentration of 20%. When the mother liquor was removed as much as possible by subjecting the slurry to a high level of filter press subsequently, the yield of a concentrated product with a 2, 6-DMN content of 90% declined to 18.9 kg.

This concentrated product was crushed, and after adding benzene measuring twice the weight of the concentrated product to it, a resultant mixture was agitated at 20°C for a period of one hour. Crystals having a 2, 6-DMN purity of 99% obtained by subsequently performing solid-liquid separation using a centrifugal separator weighed 12.2 kg (30.6% of the amount of 2, 6-DMN contained in the feed stock) The recovery of 2, 6-DMN was only about 68% of that achieved in practical examples 1 and 2, and about 61% of that achieved in practical example 3. This means that this method is remarkably unsatisfactory in terms of the recovery of the desired substance.

### ADVANTAGEOUS EFFECTS OF THE INVENTION

Configured as illustrated in the foregoing discussion, the present invention makes it possible to recover a particular crystalline substance of high purity in high yield from feed stock containing it by relatively easy operation by a skillful combination of concentration by pressure crystallization and purification by cooling crystallization, or purification by rinsing.

## Claims

1. A method for purifying a particular substance from a mixture containing the same, said method being characterized by comprising steps (1) and (2a) stated below:
(1) subjecting said mixture in the form of a liquid or a slurry in which part of said particular substance has crystallized to crystallization operation under high pressure to increase the amount of crystals of said particular substance to thereby obtain crystals enriched with said particular substance; and
(2a) dissolving the crystals enriched with said particular substance obtained in the above step in a solvent capable of well dissolving the crystals and subjecting a resultant solution to cooling crystallization to recrystallize said particular substance.

2. A method according to claim 1, wherein the solvent is removed off from mother liquor left after separating said particular substance formed by recrystallization in step (2a) and a resultant residue is supplied for use in steps (1) and (2a).

3. A method according to claim 2, wherein the solvent removed from the mother liquor is reused as at least part of the solvent used in step (2a).

4. A method for purifying a particular substance from a mixture containing the same, said method being characterized by comprising steps (1) and (2b) stated below:
(1) subjecting said mixture in the form of a liquid or a slurry in which part of said particular substance has crystallized to crystallization operation under high pressure to increase the amount of crystals of said particular substance to thereby obtain crystals enriched with said particular substance; and
(2b) rinsing the crystals enriched with said particular substance obtained in the above step with a solvent capable of dissolving the crystals.

5. A method according to claim 4, wherein the solvent is removed off from a rinsing solution obtained by rinsing the crystals of said particular substance in step (2b) and a resultant residue is supplied for use in steps (1) and (2b).

6. A method according to claim 5, wherein the solvent removed from the rinsing solution is reused as at least part of the solvent used in step (2b).

7. A method according to claim 1 or 4, wherein said particular substance is 2, 6-dimethylnaphthalene and said mixture contains an isomer of dimethylnaphthalene.

8. A method according to claim 7, wherein the solvent capable of well dissolving the crystals includes an alcohol.

9. A method according to claim 7, wherein the solvent capable of well dissolving the crystals is an aromatic solvent.

10. A method according to claim 1 or 4, wherein the pressure applied during said crystallization operation in step (1) is set within a range of 500 kg/cm² to 3,000 kg/cm².
